# EUROPEAN PATENT APPLICATION

(11) **EP 4 155 381 A1**
(43) Date of publication of application: **29.03.2023**
(21) Application number: 21809615.4
(22) Date of filing: 19.05.2021
(51) Int. Cl.: C12N 1/16, C12G 3/024

(54) **CULTURE MEDIUM ADDITIVE FOR YEAST**

(30) Priority: 22.05.2020 JP 2020089607
(71) Applicant: Asahi Group Holdings, Ltd., Tokyo 130-8602 (JP)
(72) Inventor: ARAI Marina, Moriya-shi, Ibaraki 302-0106 (JP); KAWAGUCHI Kyosuke, Moriya-shi, Ibaraki 302-0106 (JP); SHIOZAWA Tadasu, Moriya-shi, Ibaraki 302-0106 (JP); MIZUNO Megumi, Moriya-shi, Ibaraki 302-0106 (JP)
(74) Representative: Hoffmann Eitle
(86) International application number: PCT/JP2021/018906
(87) International publication number: WO 2021/235466

(57) **Abstract**

The invention provides a culture medium additive for yeast that contains mango juice as an active ingredient, a method for proliferating a yeast by proliferating the yeast in a culture medium containing the culture medium additive for yeast, and a method for fermenting a yeast by fermenting the yeast in a culture medium containing the culture medium additive for yeast.

## Description

### [Technical Field]

The present invention relates to an additive containing a plant-based material as an active ingredient that is used for promoting the fermentation and/or proliferation of a yeast, and also relates to a method for proliferating or fermenting a yeast by culturing the yeast in a culture medium containing the additive.

### [Background Art]

Fermentation has been widely used in food processing since long ago. Examples include fermented liquors such as beer and wine, and fermented foods that utilize a yeast or lactic acid bacteria such as fermented milk, natto and miso. These fermented foods contain all manner of components produced by microorganisms as a result of the fermentation, and in recent years, the health effects of these components are attracting much attention. Further, the microorganisms themselves are also being used as food materials. Examples include supplements and the like containing yeast extracts or actual lactic acid bacteria that are expected to assist in the regulation of intestinal function.

In the production of processed foods that utilize actual microorganisms or the fermentation caused by microorganisms, from the viewpoint of improving the production efficiency, it is desirable to promote the proliferation or fermentation of the microorganisms. The most widely used promoters for microorganisms, particularly in the case of the proliferation or fermentation of yeast, are mainly peptones and yeast extracts and the like. However, as a result of the growing preferences for healthy alternatives in recent years, the use of natural materials, and particularly plant-based materials, for promoting the proliferation of microorganisms for foods is becoming increasingly desirable.

Examples of plant-based materials that have been reported as promoting the proliferation or fermentation of yeast include an Araliaceae ginseng saponin-containing product (Patent Document 1) and a malt root extract (Patent Document 2). Further, the fruit or extracts of plants of the genus Ananas are known as proliferation promoters for lactic acid bacteria (Patent Document 3).

### [Citation List]

### [Patent Documents]

[Patent Document 1]
   Japanese Unexamined Patent Application, First Publication No. 2016-111967
[Patent Document 2]
   Japanese Unexamined Patent Application, First Publication No. 2009-161448
[Patent Document 3]
   Japanese Patent (Granted) Publication No. 5750785

### [Summary of Invention]

### [Technical Problem]

Although promoters for the proliferation and/or fermentation of yeast formed from plant-based materials are keenly sought for the production of foods made from natural materials, no material has yet been found that satisfactorily promotes the proliferation or fermentation of yeast.

The present invention has the objects of providing a culture medium additive for yeast that contains a plant-based material as an active ingredient and can be used for promoting the fermentation or proliferation of yeast, and also providing a method for cultivating yeast in a culture medium containing the additive.

### [Solution to Problem]

The inventors of the present invention discovered that by conducting the cultivation of yeast in the presence of mango juice, the proliferation or fermentation of the yeast could be promoted, thus enabling them to complete the present invention.

In other words, the present invention is as described below.
[1] A culture medium additive for yeast, containing mango juice as an active ingredient.
[2] The culture medium additive for yeast according to [1] above, wherein the mango juice is a reconstituted juice from a concentrate.
[3] The culture medium additive for yeast according to [1] or [2] above, wherein the mango juice is a transparent juice.
[4] The culture medium additive for yeast according to any one of [1] to [3] above, which is used to promote the fermentation of the yeast.
[5] The culture medium additive for yeast according to any one of [1] to [3] above, which is used to promote the proliferation of the yeast.
[6] The culture medium additive for yeast according to [4] or [5] above, wherein the yeast is a fungus of the genus Saccharomyces.
[7] The culture medium additive for yeast according to [6] above, wherein the fungus of the genus Saccharomyces is Saccharomyces cerevisiae.
[8] A method for cultivating a yeast by cultivating the yeast in a culture medium containing the culture medium additive for yeast according to any one of [1] to [7] above.
[9] The method for cultivating a yeast according to [8] above, wherein the culture medium contains a fruit juice besides the mango juice.
[10] A method for cultivating a yeast by cultivating the yeast in mango juice.
[11] A method for fermenting a yeast by fermenting the yeast in a culture medium containing the culture medium additive for yeast according to any one of [1] to [7] above.
[12] The method for fermenting a yeast according to [11] above, wherein the culture medium contains a fruit juice besides the mango juice.
[13] A method for fermenting a yeast by fermenting the yeast in mango juice.
[14] A fermented fruit juice obtained by fermenting a fruit juice containing mango juice with a yeast.
[15] The fermented fruit juice according to [14] above, wherein the fruit juice is a reconstituted juice from a concentrate.
[16] A beverage containing the fermented fruit juice according to [14] or [15] above.

### [Advantageous Effects of Invention]

The culture medium additive for yeast according to the present invention contains a plant-based mango juice as an active ingredient, and has the effects of promoting the proliferation or fermentation of yeast. Accordingly, the culture medium additive for yeast according to the present invention is useful in the production of processed food and beverages, or the raw materials therefor.

### [Brief Description of Drawings]

FIG. 1 is a diagram illustrating the results, in Example 3, of inoculating and cultivating mango juice, pineapple juice and apple juice with the Saccharomyces cerevisiae strain NCYC917, and measuring the amount of alcohol produced (vol%) over time.
FIG. 2 is a diagram illustrating the results, in Example 3, of inoculating and cultivating mango juice, pineapple juice and apple juice with the Saccharomyces cerevisiae strain NCYC917, and measuring the turbidity (OD660) over time.

### [Description of Embodiments]

The culture medium additive for yeast according to the present invention contains mango (Mangifera indica) juice as an active ingredient. The reasons why a yeast proliferation promotion effect or fermentation promotion effect is achieved when a yeast is cultivated in the presence of mango juice are not entirely clear, but it is thought that there is a possibility that mango juice may contain a component that contributes to the promotion of proliferation or fermentation of yeast.

The mango juice that represents the active ingredient of the culture medium additive for yeast according to the present invention is a juice obtained by juice extraction from the mango flesh. There are no particular limitations on the variety or the production region or the like of the mango used as the raw material for the mango juice used in the invention, and any of the many varieties typically supplied as raw fruit or as processed foods may be used.

The mango juice used as the raw material for the culture medium additive for yeast according to the present invention may be a juice obtained by crushing the mango flesh and either extracting the juice or using the puree, and then removing the skin and the seeds and the like (a mango juice extraction), a juice obtained by concentrating a mango juice extraction (a concentrated juice), a juice obtained by diluting a concentrated juice (a reconstituted juice), or a juice in which a portion of insoluble components have been removed from one of the above juices.

There are no particular limitations on the method used for preparing the mango juice from the raw material mangoes, and the type of processing methods typically used during juice preparation (methods involving an appropriate combination of operations such as washing, selection, crushing, juice extraction, separation, heating and cooling) may be employed. For example, the juice can be produced using the methods typically employed for producing the raw material fruit juices used in fruit juice beverages, such as a method in which the entire fruit (including the skin and the like) is squeezed and separated into a juice component and a residue containing the skin using an inline juice extractor or the like, and this squeezed and separated juice is then concentrated, sterilized and cooled, or a method in which the whole fruit is cut in half, the juice is then extracted only from the fruit flesh, and the resulting juice is then concentrated, sterilized and cooled.

The mango juice used as the raw material for the culture medium additive for yeast according to the present invention is preferably a transparent juice from which at least a portion of the insoluble solid matter such as the pulp has been separated and removed. Examples of the method used for separating and removing the insoluble solid matter include methods such as centrifugal separation, diatomaceous earth filtration and membrane separation, but centrifugal separation is particularly desirable.

The obtained mango juice may, if necessary, be concentrated to a Brix of at least 20% by mass but not more than 70% by mass using evaporation concentration, freeze concentration, or reverse osmosis membrane concentration or the like, and this concentrated juice may be used either as is, or following dilution with drinking water or the like to an appropriate concentration, as the main component of the culture medium additive for yeast according to the present invention. Further, in those cases where there is time before the mango juice is to be supplied for use as a raw material for the culture medium additive for yeast, the juice may be sterilized, subsequently used to fill a polyethylene bag or the like under sterile conditions, and then stored by refrigeration or freezing. From the perspective of storage and handleability and the like, a concentrated juice is preferred. The sterilization processing may employ any method selected appropriately from the various methods used in the sterilization of fruit juices and processed products thereof, including heat sterilization and filter sterilization.

In the present invention and in this description, the Brix value of the mango juice can be measured by normal methods using a measuring device such as a refractometer (RX-5000α, manufactured by ATAGO Co., Ltd.).

The culture medium additive for yeast according to the present invention may be composed solely of mango juice, or may also contain various other additives in addition to the mango juice, provided these additives do not impair the yeast proliferation promotion action or fermentation promotion action. These additives may be selected appropriately from among antioxidants such as ascorbic acid and sodium ascorbate; pH modifiers including inorganic acids, organic acids, and salts thereof; and the various types of additives typically added to fruit juices and fruit juice processed products such as flavorings.

The culture medium additive for yeast according to the present invention is added to a culture medium used for cultivating the yeast. There are no particular limitations on the yeast cultivating medium to which the culture medium additive for yeast according to the present invention is added, and the types of culture media used in normal methods may be used. For example, one or more media selected from the group consisting of glucose, which is used as the carbon source in the cultivation of most typical microorganisms, sucrose, acetic acid, ethanol, molasses and spent sulfite pulp liquor and the like may be used. As a nitrogen source, one or more compounds selected from the group consisting of urea, ammonia, inorganic salts such as ammonium sulfate, ammonium chloride and ammonium phosphate, and nitrogen-containing organic substances such as corn steep liquor (CSL), casein, yeast extracts and peptones may be used. Moreover, phosphoric acid components, potassium components and magnesium components may also be added to the culture medium, and these components may be typical industrial raw materials such as calcium superphosphate, ammonium phosphate, potassium chloride, potassium hydroxide, magnesium sulfate and magnesium hydrochloride. In addition, inorganic salts of zinc, copper, manganese and iron ions and the like may also be used. Vitamins and the like may also be added. Specifically, the types of culture media generally used in yeast cultivation such as YPD liquid media and SD media may be used.

In those cases where the culture medium additive for yeast according to the present invention is used for promoting the proliferation or fermentation of a yeast used in the production of a food or beverage, or a yeast used as a material for a food or beverage, the yeast culture medium to which the culture medium additive for yeast according to the present invention is added is preferably a culture medium in which the main carbon sources and/or nitrogen sources are composed of natural materials. Of the various possibilities, a culture medium containing a fruit juice or vegetable juice other than mango juice is preferred, a culture medium in which a fruit juice or vegetable juice constitutes the main carbon source and/or nitrogen source (hereinafter sometimes referred to as a "fruit juice culture medium") is more preferred, and a fruit juice or vegetable juice itself may be used, and mango juice itself may also be used. By proliferating and fermenting the yeast in a fruit juice culture medium, a fermented fruit juice or fermented vegetable juice, or a fermented liquor such as wine or cider can be produced. In other words, by using the culture medium additive for yeast according to the present invention, the time required for the proliferation and fermentation of the yeast in the production process for a fermented fruit juice, fermented vegetable juice or fermented liquor can be shortened, enabling a more efficient production.

There are no particular limitations on the amount of the culture medium additive for yeast according to the present invention incorporated in the yeast culture medium, provided the amount is sufficient to achieve a yeast proliferation promotion effect or fermentation promotion effect. For example, the culture medium additive for yeast may be added to the yeast culture medium in sufficient amount to achieve an extract fraction derived from mango juice (a mango juice-derived Brix value) that is preferably at least 0.1% by mass but not more than 70% by mass, more preferably at least 0.5% by mass but not more than 55% by mass, and even more preferably at least 1% by mass but not more than 27% by mass. Proliferation or fermentation of the yeast may be conducted using only mango juice as the culture medium.

There are no particular limitations on the fruit juices or vegetable juices that may be included in the yeast culture medium, and juice extracts from various fruits and vegetables or processed products thereof may be used. Examples of the fruit juices include the juices of soft fruits such as strawberries, peaches, melons, grapes, apples, pears, Japanese pears and cherries; tropical fruits such as bananas, pineapples, mangoes and passionfruit; and citrus fruits such as lemons, limes, yuzu, shekwasha, sudachi, kabosu, grapefruit, oranges, iyokan, satsuma mandarins, Chinese citron, hassaku and hyuganatsu. Further examples of vegetable juices that may be used include the juice extracts of tomatoes, carrots, spinach, cabbage, broccoli, cauliflower, celery, lettuce, parsley, cress, kale, soybeans, beets, red capsicums, pumpkin and komatsuna. The fruit juice or vegetable juice incorporated in the yeast culture medium may be the fruit or vegetable juice extract itself, a concentrated fruit juice or concentrated vegetable juice, a reconstituted fruit juice or reconstituted vegetable juice, or one of these juices from which at least a portion of the insoluble solid matter has been removed. These fruit juices and vegetable juices, and the concentrated juices and reconstituted juices thereof can be prepared, for example, using methods typically employed when producing juices or raw materials therefor.

The yeast culture conditions may be any set of conditions that conform with typical yeast culture conditions, and for example, the temperature is typically within a range from 20 to 40°C, and preferably from 25 to 35°C, and the pH is typically within a range from 3.5 to 7.5, and preferably from 4.0 to 6.0.

The yeast fermentation conditions may be any set of conditions that conform with typical yeast fermentation conditions, and for example, the temperature is typically within a range from 0 to 35°C, and the pH is typically within a range from 3.0 to 7.5, and preferably from 3.5 to 6.0. The yeast proliferation or fermentation may be conducted under aerobic conditions or anaerobic conditions. Furthermore, either a static culture of a stirred culture may be conducted.

The yeast that is cultivated in the presence of the culture medium additive for yeast according to the present invention may be any single-cell eumycete, and specific examples include fungi of the genus Saccharomyces, fungi of the genus Schizosaccharomyces, fungi of the genus Pichia, fungi of the genus Candida, fungi of the genus Kluyveromyces, fungi of the genus Williopsis, fungi of the genus Debaryomyces, fungi of the genus Galactomyces, fungi of the genus Torulaspora, fungi of the genus Rhodotorula, fungi of the genus Yarrowia, fungi of the genus Zygosaccharomyces, fungi of the genus Hanseniaspora, fungi of the genus Cyberlindnera, and fungi of the genus Brettanomyces (Dekkera). Among these, because they are edible, Saccharomyces cerevisiae, Saccharomyces pastorianus, Pichia anomala, Pichia (Komagataella) pastoris, Candida tropicalis, Candida lypolitica, Candida sake, Kluyveromyces lactis, Kluyveromyces marxianus, Debaryomyces hansenii, Hanseniaspora uvarum, Cyberlindnera jadinii, and Brettanomyces (Dekkera) bruxellensis and the like are preferred, and the commonly used Saccharomyces cerevisiae is particularly preferred.

The yeast cells obtained by cultivating an edible yeast in the presence of the culture medium additive for yeast according to the present invention may be used, in a similar manner to other edible yeasts, as a raw material for all manner of beverages and foods. Further, a fermented product obtained by fermenting an edible yeast in the presence of the culture medium additive for yeast according to the present invention may also be used in all manner of beverages and foods. For example, fermented liquors such as wine and cider obtained by conducting fermentation in the presence of the culture medium additive for yeast according to the present invention may be used as beverages without further modification, or may be used as a raw material for blended liquors. Further, fermented fruit juices and fermented vegetable juices obtained by conducting fermentation in the presence of the culture medium additive for yeast according to the present invention may also be used as beverages without further modification, or may be used as a raw material for other beverages or foods.

### [Examples]

The present invention is described below in further detail using a series of examples, but the present invention is not limited to the following examples.

### <Juice Preparation>

Unless specifically stated otherwise, the fruit juices used in the following tests were reconstituted juices formed from concentrates. Specifically, commercially available reconstituted juices from concentrates were prepared with a target Brix value, and then subjected to filter sterilization prior to use.

### <Preparation of Starter>

In the following tests, unless specifically stated otherwise, preculturing of the yeast being used was conducted in the following manner. First, a YPD liquid culture medium was inoculated with the yeast, and a shaking culture was conducted at 25°C for 48 hours. Subsequently, the yeast cells were recovered by a centrifugal separation treatment (7,000×g, 5 minutes) and washed with sterilized water, and a starter was then prepared by adjusting the cell concentration to 1.0 × 10⁸ cells/mL with sterilized water.

### <Measurement of Yeast Degree of Fermentation>

In the following tests, unless specifically stated otherwise, the degree of fermentation of the yeast was determined using the amount of alcohol produced as an indicator. The amount of alcohol in a sample was determined by measuring a solution prepared by diluting the sample with distilled water to achieve an alcohol concentration within a range from 0.0 to 2.0 vol% with a multifunctional biosensor (BF-5D, manufactured by Oji Scientific Instruments Co., Ltd.). This multifunctional biosensor is a device that electrochemically detects the hydrogen peroxide (H₂O₂) produced as a result of oxidation by alcohol oxidase. The value obtained by multiplying the obtained measurement value by the dilution factor was deemed to be the alcohol concentration (vol%) within the sample.

### <Measurement of Yeast Degree of Proliferation>

In the following tests, unless specifically stated otherwise, the degree of proliferation of the yeast was determined using the turbidity (OD660: the absorbance at 660 nm) as an indicator. The OD660 value was measured using a turbidity meter (UV-1800, manufactured by Shimadzu Corporation).

### [Example 1]

In order to extract a fruit juice in which the fermentation of yeast proceeds favorably, the degree of fermentation was compared in various fruit juice culture media. A total of nine types of fruit juice were used as the fruit juice culture media, namely a mango juice 1 (a freeze concentrated mango transparent juice <MEXIFRUTAS> of the Tommy Atkins variety, manufactured by Mexifrutas S.A. de C.V.), a mango juice 2 (a mango transparent concentrated juice of the Carabao variety, manufactured by KLT Fruits, Inc.), a pineapple juice (a golden pineapple 5-fold concentrated transparent juice, manufactured by Kakoh Co., Ltd.), an apple juice (a freeze concentrated apple transparent juice, manufactured by Dalian Haisheng Fresh Fruit Juice Co., Ltd.), a red grape juice (68° grape transparent high-polyphenol juice, manufactured by Welch Foods Inc.), a white grape juice (68° white grape transparent juice, manufactured by ENAV S.A.), a La France pear juice (a Yamagata La France 5-fold concentrated transparent juice, manufactured by Kakoh Co., Ltd.), a kiwifruit juice (a freeze concentrated kiwifruit transparent juice, manufactured by ProFruit 2006 Limited), and a muscat juice (a Z10 muscat transparent concentrated juice, manufactured by Australian Vintage Limited). The fruits juices used were all prepared with a Brix value of 10% by mass in accordance with the above section entitled <Juice Preparations

A 10 mL sample of each fruit juice was inoculated with the Saccharomyces cerevisiae NCYC917 strain, prepared in accordance with the above section entitled <Preparation of Starter>, in sufficient amount to achieve a concentration of 1% (1.0 × 10⁶ cells/mL), and the resulting sample was then subjected to static culture at 25°C for 24 hours. Following the culture, the cultured product was subjected to filter sterilization, and the amount of alcohol produced was measured.

The measurement results for the amounts of alcohol produced in the obtained fermented fruit juices are shown in Table 1. The results indicated that the mango juice 1 culture medium and the mango juice 2 culture medium yielded the greatest amounts of alcohol produced, indicating favorable levels of fermentation.

**[Table 1]**

| Test Sample No. | Raw material fruit juice | Amount of alcohol produced [vol%] |
|---|---|---|
| 1 | Mango 2 | 0.918 |
| 2 | Mango 1 | 0.912 |
| 3 | Pineapple | 0.572 |
| 4 | Red grape | 0.400 |
| 5 | La France | 0.319 |
| 6 | Apple | 0.163 |
| 7 | Kiwifruit | 0.091 |
| 8 | White grape | 0.029 |
| 9 | Muscat | 0.026 |

### [Example 2]

Testing was conducted to confirm that the fermentation promotion effect provided by mango juice could be achieved with any Saccharomyces cerevisiae regardless of the strain. For the Saccharomyces cerevisiae, a total of seven strains were used, including the NCYC917 strain (held by the National Collection of Yeast Culture) used in Example 1, as well as the Maurivin SW strain (held by Mauri, Inc.), and the NBRC0848 strain, the NBRC0849 strain, the NBRC0850 strain, the NBRC0853 strain, and the NBRC0965 strain (all held by the National Institute of Technology and Evaluation Biotechnology Center). These strains were selected from among the various strains procurable from microorganism collection organizations or distributors, selecting those strains that are typically used as wine yeast in fermentation, or those strains that can be used in fermentation using grapes or Japanese sake as the separation source.

Using the mango juice 1, the pineapple juice and the apple juice used in Example 1 as fruit juice culture media, each culture medium was inoculated with each of the above strains in the same manner as that described in Example 1, and in each case the resulting mixture was then fermented, and the amount of alcohol produced was measured. The measurement results are shown in Table 2. These results revealed that for all of the yeast strains, the amount of alcohol produced was greatest and the fermentation was superior in the mango juice culture medium.

**[Table 2]**

| Strain | Raw material fruit juice | Amount of alcohol produced [vol%] |
|---|---|---|
| NCYC917 | Mango | 0.649 |
| | Pineapple | 0.459 |
| | Apple | 0.140 |
| Maurivin SW | Mango | 1.622 |
| | Pineapple | 1.404 |
| | Apple | 1.270 |
| NBRC0848 | Mango | 1.950 |
| | Pineapple | 1.312 |
| | Apple | 0.770 |
| NBRC0849 | Mango | 1.496 |
| | Pineapple | 1.396 |
| | Apple | 1.114 |
| NBRC0850 | Mango | 1.350 |
| | Pineapple | 0.972 |
| | Apple | 0.894 |
| NBRC0853 | Mango | 1.846 |
| | Pineapple | 1.446 |
| | Apple | 1.278 |
| NBRC0965 | Mango | 1.734 |
| | Pineapple | 1.552 |
| | Apple | 1.310 |

### [Example 3]

The degree of proliferation and the degree of fermentation of Saccharomyces cerevisiae in mango juice, pineapple juice and apple juice were measured over time and compared.

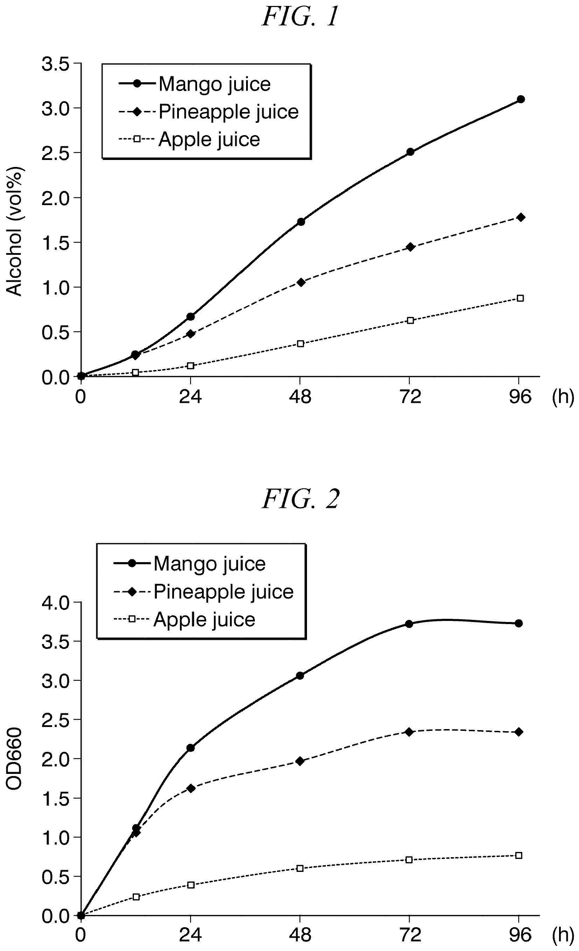

Specifically, the mango juice, the pineapple juice and the apple juice used in Example 2 were each inoculated with the NCYC917 strain used in Example 1 in the same manner as that described in Example 1, samples were extracted at 0, 12, 24, 48, 72 and 96 hours after starting the culture, and the amount of alcohol produced (vol%) and the turbidity (OD660) were measured for each sample. The measurement results for the amount of alcohol produced are illustrated in FIG. 1, and the measurement results for the turbidity are illustrated in FIG. 2.

As illustrated in FIG. 1 and FIG. 2, compared with those cases where the pineapple juice or the apple juice was used as the culture medium, when the mango juice was used as the culture medium the turbidity was highest and the amount of alcohol produced was greatest, from 12 hours after until at least 96 hours after the start of the culture. These results indicated that mango juice promotes both the proliferation and the fermentation of yeast to a greater extent than other fruit juices.

### [Example 4]

The effects of the inoculation conditions and the temperature conditions on the culture of yeast in the presence of mango juice were investigated.

Specifically, with the exceptions of altering the amount of yeast with which the mango juice was inoculated to either 1.0 × 10⁵ cells/mL or 1.0 × 10⁶ cells/mL, and setting the culture temperature to either 25°C or 30°C, the mango juice, the pineapple juice and the apple juice were each inoculated with the NCYC917 strain and then cultured in the same manner as Example 2, and following the culture, the amount of alcohol produced and the turbidity (OD660) were measured.

**[Table 3]**

| Test No. | Initial cell count [cells/mL] | Culture temperature [°C] | Raw material fruit juice | Amount of alcohol produced [vol%] | Turbidity (OD660) |
|---|---|---|---|---|---|
| 1 | 1.0E+05 | 25 | Mango | 0.359 | 1.830 |
| 2 | | | Pineapple | 0.297 | 1.515 |
| 3 | | | Apple | 0.072 | 0.274 |
| 4 | 1.0E+05 | 30 | Mango | 0.678 | 2.315 |
| 5 | | | Pineapple | 0.459 | 1.810 |
| 6 | | | Apple | 0.125 | 0.361 |
| 7 | 1.0E+06 | 25 | Mango | 0.681 | 2.365 |
| 8 | | | Pineapple | 0.466 | 1.760 |
| 9 | | | Apple | 0.129 | 0.419 |
| 10 | 1.0E+06 | 30 | Mango | 0.946 | 2.500 |
| 11 | | | Pineapple | 0.534 | 1.795 |
| 12 | | | Apple | 0.182 | 0.506 |

The measurement results are shown in Table 3. These results revealed that regardless of the culture conditions, the amount of alcohol produced was greatest, indicating the fermentation had progressed the most, in the mango juice culture medium. In a similar manner, regardless of the culture conditions, the turbidity was highest, indicating superior proliferation of the yeast, in the mango juice culture medium.

### [Example 5]

The fermentation promotion effect obtained by adding mango juice to a yeast culture medium was investigated. The apple juice, the white grape juice and the red grape juice used in Example 1 were used as the raw material fruit juices for the fruit juice culture medium. The mango juice 1, the pineapple juice and the apple juice used in Example 1 were used as fruit juices for adding to the fruit juice culture medium as additives (namely, additive fruit juices).

Specifically, first, the raw material fruit juice used as the fruit juice culture medium was prepared in accordance with the above section entitled <Juice Preparation> so as to achieve a final Brix value of 10% by mass. On the other hand, the mango juice, pineapple juice and apple juice used as the additive fruit juices were prepared and added to the fruit juice culture medium so as to achieve a final Brix value derived from the additive in the fruit juice culture medium of 0.5% by mass of 1 % by mass. Further, as a control, an amount of ultrapure water equal to the volume of the added additive fruit juices was added to the culture medium.

A 10 mL sample of each of the fruit juice culture media to which an additive fruit juice had been added was inoculated with the Saccharomyces cerevisiae NCYC917 strain, prepared in accordance with the above section entitled <Preparation of Starter>, in sufficient amount to achieve a concentration of 1% (1.0 × 10⁶ cells/mL), and the resulting sample was then subjected to static culture at 25°C for 24 hours. Following the culture, the cultured product was subjected to filter sterilization, and the amount of alcohol produced was measured.

**[Table 4]**

| Test No. | Raw material fruit juice | Additive fruit juice final Brix value | Additive fruit juice | Amount of alcohol produced [vol%] |
|---|---|---|---|---|
| 1 | Apple | 0% by mass | Ultrapure water | 0.130 |
| 2 | | 0.5% by mass | Mango | 0.172 |
| 3 | | | Pineapple | 0.151 |
| 4 | | | Apple | 0.119 |
| 5 | | 1% by mass | Mango | 0.235 |
| 6 | | | Pineapple | 0.208 |
| 7 | | | Apple | 0.143 |
| 8 | White grape | 0% by mass | Ultrapure water | 0.037 |
| 9 | | 0.5% by mass | Mango | 0.087 |
| 10 | | | Pineapple | 0.072 |
| 11 | | | Apple | 0.045 |
| 12 | | 1% by mass | Mango | 0.155 |
| 13 | | | Pineapple | 0.119 |
| 14 | | | Apple | 0.038 |
| 15 | Red grape | 0% by mass | Ultrapure water | 0.320 |
| 16 | | 0.5% by mass | Mango | 0.362 |
| 17 | | | Pineapple | 0.352 |
| 18 | | | Apple | 0.318 |
| 19 | | 1% by mass | Mango | 0.443 |
| 20 | | | Pineapple | 0.383 |
| 21 | | | Apple | 0.341 |

The measurement results are shown in Table 4. The results revealed that in those fruit juice culture media in which the additive fruit juice had been added in sufficient amount to achieve a Brix value of 1 % by mass, in all of the raw material fruit juices, the amount of alcohol produced was greatest in the fruit juice culture medium to which the mango juice had been added, indicating that fermentation had been promoted by the addition of the mango juice. Further, even in those fruit juice culture media in which the additive fruit juice had been added in sufficient amount to achieve a Brix value of 0.5% by mass, fermentation was promoted the most in the fruit juice culture medium to which the mango juice had been added.

### [Example 6]

The proliferation promotion effect obtained by adding mango juice to a yeast culture medium was investigated. The yeast culture medium used was a glucose-free SD medium (0.67% Yeast Nitrogen Base w/o Amino Acids, manufactured by DIFCO) which represents a minimum nutrient medium. For the juices added as additives to the culture medium (namely, the additive juices), in addition to the mango juice 1, the pineapple juice and the apple juice used in Example 5, molasses (derived from sugar beet) and glucose were also used.

Specifically, first, the mango juice, the pineapple juice, the apple juice, the molasses and the glucose used as the additives were each prepared and added to the glucose-free SD medium so as to achieve a final Brix value derived from the additive of 1% by mass. Further, as a control, an amount of ultrapure water equal to the volume of the added additive juices was added to the culture medium.

A 10 mL sample of each of the culture media was inoculated with the Saccharomyces cerevisiae NCYC917 strain, prepared in accordance with the above section entitled <Preparation of Starter>, in sufficient amount to achieve a concentration of 1% (1.0 × 10⁶ cells/mL), and the resulting sample was then subjected to static culture at 25°C for 24 hours. Following the culture, the turbidity (OD660) of the cultured product was measured.

**[Table 5]**

| Raw material culture medium | Additive | Turbidity following fermentation (00660) |
|---|---|---|
| Glucose-free SD medium | Ultrapure water | 0.052 |
| | Mango juice | 0.570 |
| | Pineapple juice | 0.457 |
| | Apple juice | 0.159 |
| | Molasses | 0.145 |
| | Glucose | 0.100 |

The measurement results are shown in Table 5. The results revealed that the cultured product from the culture medium to which the mango juice had been added had the highest turbidity, indicating that proliferation of the yeast was promoted by the addition of the mango juice.

## Claims

1. A culture medium additive for yeast, containing mango juice as an active ingredient.

2. The culture medium additive for yeast according to Claim 1, wherein the mango juice is a reconstituted juice from a concentrate.

3. The culture medium additive for yeast according to Claim 1 or 2, wherein the mango juice is a transparent juice.

4. The culture medium additive for yeast according to any one of Claims 1 to 3, which is used to promote fermentation of the yeast.

5. The culture medium additive for yeast according to any one of Claims 1 to 3, which is used to promote proliferation of the yeast.

6. The culture medium additive for yeast according to Claim 4 or 5, wherein the yeast is a fungus of genus Saccharomyces.

7. The culture medium additive for yeast according to Claim 6, wherein the fungus of the genus Saccharomyces is Saccharomyces cerevisiae.

8. A method for cultivating a yeast by cultivating the yeast in a culture medium containing the culture medium additive for yeast according to any one of Claim 1 to 7.

9. The method for cultivating a yeast according to Claim 8, wherein the culture medium contains a fruit juice besides the mango juice.

10. A method for cultivating a yeast by cultivating the yeast in mango juice.

11. A method for fermenting a yeast by fermenting the yeast in a culture medium containing the culture medium additive for yeast according to any one of Claims 1 to 7.

12. The method for fermenting a yeast according to Claim 11, wherein the culture medium contains a fruit juice besides the mango juice.

13. A method for fermenting a yeast by fermenting the yeast in mango juice.

14. A fermented fruit juice obtained by fermenting a fruit juice containing mango juice with a yeast.

15. The fermented fruit juice according to Claim 14, wherein the fruit juice is a reconstituted juice from a concentrate.

16. A beverage containing the fermented fruit juice according to Claim 14 or 15.
